# EUROPEAN PATENT APPLICATION

(11) **EP 1 867 307 A1**
(43) Date of publication of application: **19.12.2007**
(21) Application number: 07447038.6
(22) Date of filing: 18.06.2007
(51) Int. Cl.: A61F 5/01

(54) **A dynamic ankle orthesis**

(30) Priority: 16.06.2006 EP 06012438
(71) Applicant: Ortec N.V., 3001 Leuven (BE)
(72) Inventor: Devreese, Serge Lucien Pierre Michel, 4910 Theux (BE)
(74) Representative: Bird, Ariane

(57) **Abstract**

A dynamic ankle orthesis is provided, which dynamic ankle orthesis comprises
- a cuff for being fit to the lower part of a leg;
- a means for fitting a foot, the means having a longitudinal direction being aligned with the longitudinal direction (A*) of the foot;
- a joint suitable for being located behind the heel;
- a first coupling means, which couples the cuff to the joint;
- a second coupling means which couples the means for fitting a foot to the joint;
- an ankle-cuff to be fit to a leg above the ankle and located between joint and the cuff for being fit to the lower part of a leg, the ankle-cuff being coupled to the first coupling means;
- at least a first elastic tensioning means, the at least the first elastic tensioning means coupling the means for fitting a foot to the ankle-cuff.

## Description

### Technical field of the invention

The present invention relates to a dynamic ankle orthesis, especially an orthesis for maintaining the dynamical behaviour of the combination of foot-ankle-leg, and for assisting and/or correcting neuromuscular deficiencies related to one or more of the three rotation axes of the foot. Rotations corresponding to these three rotation axes are enabling a rotation around the vertical axis of the leg, stretching and bending of the ankle around the axis of the ankle and supination/pronation, this is rotation around the longitudinal axis of the foot.

### Background of the invention

A normal step of a person comprises several consecutive phases. In a first phase, called "heel-strike phase", the heel is placed on the ground under a slight supination, this is the foot being slightly inclined to the exterior side of the foot. The contact of the foot with the ground is made at the external back part of the heel.

In a second phase, called "mid-stance phase", the sole of the foot is brought in contact with the ground. The foot is canted progressively forward meanwhile making a pronation, this is rotating the foot to the internal side of the foot around the longitudinal axis of the foot. This is done in preparation of the third phase, called "toe-off phase", during which the foot is pushed off at the metatarsus of the big toe. After this toe-off phase, there is a fourth phase during which the leg is stretched and the foot is swung forwards. This phase is called the swing-phase. After a swing-phase, again a heel-strike phase is exercised.

Some patients suffer from pathology like multiple sclerosis, hemiplegics, myopathy or traumatic repercussions, generating neuromuscular problems, causing an insufficient control of positioning, and maintaining of its position, of the foot and the leg during walking. In order to obtain a stabilised walk, a good neuromuscular coordination is necessary, to obtain a correct foot contact with and positioning on the ground, which is continuously changing during each phase of a step.

Existing textile ankle supports are of little or no use for this type of pathology.

Other light apparatuses consist of a leaf spring and an upholstery or semi-rigid shell, which only provided a weak support to the foot.

The majority of ortheses for providing a strong foot support are rigid or may provide an articulation around the axis of the ankle, possibly with a system of abutment points or with articulating assistance as described in JP9206347.

The articulation may be provided by a leaf spring which has a dynamical effect, as described in WO 02/096328.

In 1965, Freeman et al. first postulated and provided evidence that a proprioceptive defect, e.g. after ankle sprain, caused functional instability of the joint. Freeman et al. explained that ligaments contain nerve endings that inform the brain of joint position. Proprioceptive awareness is important in preventing re-injury by providing good sensory feedback. Immobilization of the joint results in deterioration of proprioceptive function. More generally, and not limited to physical injury such as a sprain, the non-respecting of the mobility of the ankle according to all three rotation axes, generates a loss of proprioception, this is a loss of perception of the contact of foot with the ground, which perception is to be communicated to the brain for dosing the nervous influx to the muscle or group of muscles. In addition, this loss of perception, combined with a stringent positioning of the foot wearing the orthesis, may cause spasms and unbalancing of the patient.

An orthesis articulating along a multitude of planes is shown in WO04/008987. W004/008987 describes a dynamic ankle orthesis allowing a rotation along a plurality of axes. A mortar situated behind the heel enforces a pressure, which causes a lift at the front part of the foot.

Also the document WO99/66868 describes a dynamic ankle orthesis, which allows a rotation around a plurality of axes. A system of elastic tensioners enforces traction for lifting the front part of the foot.

The last two documents provide rotation around several axes for assisting dynamically the lifting of the foot.

### Summary of the invention

It is an object of the present invention to provide an improved dynamic ankle orthesis, to be worn on a foot.

It is an object of embodiments of the present invention to harmonize the different phases in making a step. This harmonization of the different consecutive phases improves the stability of the patient and has a direct influence on the dynamic behavior of the whole body of the patient. Amongst others, it permits to ease the articulations of knees, hips and rachis, all promoting a better energetic balance.

The above objective is accomplished by a dynamic ankle orthesis according to the present invention. The orthesis may allow control and assistance of the foot during rotation around one or more, even around each of the three axes of rotation in a distinct way during each of the phases of a step.

The dynamic ankle ortheses according to the present invention provide conditioning of a spastic foot or drop foot. The dynamic ankle ortheses may stabilise the step and may provide elongation of the triceps of the leg being in permanent tension causing the foot to be oriented downward permanently. The dynamic ankle ortheses may be used to gradually elongate the triceps in permanent traction, thereby reducing the tendency of the foot to be oriented downwards. The dynamic ankle ortheses may be used as revalidation tools which can be adapted according to the spasticity and muscle tonicity of the foot of the patient during revalidation.
According to a first aspect of the present invention, a dynamic ankle orthesis is provided, which dynamic ankle orthesis comprises:
- a cuff suitable for receiving the calf of the leg of a patient;
- a means for fitting a foot;
- a joint comprising a first axis of rotation (C), the joint comprising
   o a cylindrical housing having an upper side and a lower side, the lower side being provided with an aperture, the housing having an inner surface, the housing having an cylinder axis which is aligned with the first axis of rotation,
   o a bar having a substantially cylindrical first outer end, the first outer end of the bar being rotatably mounted in the cylindrical housing via the aperture, the mounting allowing rotation of the bar around the first axis of rotation;
- a first coupling means, which couples the cuff to the upper side of the cylindrical housing;
- a second coupling means which couples the means for fitting a foot to the bar.

The inner surface of the cylindrical housing is provided with a conical shape having its widest diameter at the lower surface, said conical shape allowing the bar to swing over an angle β around at least a second axis of rotation.

The axis C is an axis substantially parallel to the vertical axis of the leg. The second axis (A) is optionally an axis substantially perpendicular to the first axis. The second axis of rotation may be aligned with the longitudinal direction of the means for fitting a foot.

A means for fitting a foot may have a longitudinal direction being aligned with the longitudinal direction (A*) of the foot. The means for fitting a foot may be a shoe-inlay, intended to contact the sole of the foot only, i.e. contacting the sole partially or completely. The means for fitting a foot may also be a shoe-inlay, intended to contact the sole of the foot, i.e. contacting the sole partially or completely, as well as a part or the complete heel part of the foot. As an other alternative, the means for fitting a foot may be shoe.

The bar may be cylindrical at its first outer end only, i.e. having a cylindrically shaped first outer end, suitable to rotate the bar in the housing. Alternatively the bar may have a cylindrical volume along its length.

The dimensions of the bar and the aperture via which the bar extends into the cylindrical housing, may be adapted to prevent the bar to substantially move in the housing in a direction parallel to the axis C.

The first coupling means may be a bar, optionally comprising ap plurality of posts, such as two posts. The first coupling means may be provided from metal, such as steel, or may be provided from polymer material, such as reinforced polymer material, e.g. glass fiber of carbon reinforced polymer material, also referred to as composite material. The first coupling means may be a rigid coupling means or a resilient means.

The second coupling means may be a resilient means, provided from any suitable material, such as e.g. metal, steel, e.g. resilient steel, polymer material, such as reinforced polymer material, e.g. glass fiber of carbon reinforced polymer material or any other suitable material.

According to some embodiments of the present invention, the joint further may comprise a third axis of rotation (B), which third axis of rotation is perpendicular to the plane defined by the first axis of rotation and the second axis of rotation. The joint may comprise a means to allow rotation of the bar around the third axis of rotation over an angle γ. Optionally, γ may be larger than β.

The conical shape of the inner surface of the housing and the cylindrical shape of the bar rotatably moveable inside the housing, may easily facilitate to provide a rotation about both the second axis A and third axis B.

The dynamic ankle ortheses according to the first aspect of the present invention provide at least some freedom to rotate about at least two, and optionally more than two, axes of rotation. This freedom allows improving the proprioception.

The coupling of the means for fitting a foot to the joint by means of a resilient coupling means, i.e. a resilient second coupling means, may provide a more dynamic foot lifting during swing-phase. This because energy obtained during the mid-stance phase, which is so-to-say 'stored' in the second coupling means, may be used to provide a propulsion following the toe-off phase. This effects a more dynamic toe-off phase, hence a more dynamic walking movement.

According to some embodiments of the present invention, at least one of the angles β or γ can be set by means of at least one setscrew . According to some embodiments of the present invention, the at least one setscrews may extend through the cylindrical housing. Optionally more than one, such as two, three or four set screws may be provided for setting the angles β or γ. The set screws may be provided as pair of set screws, the two screws of the pair of screws being mounted diametrically opposite on to the other along the surface of the cylindrical housing. The set screw or set screws may be used to limit the more dynamic foot lifting during swing-phase This freedom to rotate about 3 axes, i.e. an axis aligned with the axis of the ankle, an axis in longitudinal direction of the means for fitting a foot, hence aligned with the longitudinal direction of the foot, and the axis C, allows improving of the proprioception.

According to some embodiments of the present invention, the dynamic ankle orthesis may comprise an ankle-cuff to be fit to a leg above the ankle and located between joint and the cuff for being fit to the lower part of a leg. The ankle-cuff may be coupled to the first coupling means. The dynamic ankle orthesis may comprise at least a first elastic tensioning means, coupling the means for fitting a foot to the ankle-cuff.

According to some embodiments of the present invention, the means for fitting a foot may have a first side and a second side in longitudinal direction. The ankle-cuff may have a first side to be located at one side of a leg and a second side to be located at the other side of a leg. The orthesis may comprise a first and a second elastic tensioning means, the first elastic tensioning means coupling the first side of the means for fitting a foot to the first side of the ankle-cuff, the second elastic tensioning means coupling the second side of the means for fitting a foot to the second side of the ankle-cuff.

According to some embodiments of the present invention, each of the elastic tensioning means may comprises a elliptic-shaped elastic tensor and a first and a second coupling means for providing tension to the tensor in a direction along the long axis of the ellipse. The elastic tensioning means may comprise at least one extension limiting means coupled to a first and a second coupling means for limiting the dilatation of first and a second coupling means. According to some embodiments of the present invention, the elastic tensioning means further may comprise at least one abutment located between a first and a second coupling means for limiting the approximation of first and a second coupling means. The abutments may optionally be adjustable. The limitation of the elastic contracting of the elastic tensioning means allows to adjust the backwards flexion of the foot, i.e. the bending of the ankle.

According to a second aspect of the present invention, a dynamic ankle orthesis is provided, which dynamic ankle orthesis comprises
- a cuff for being fit to the lower part of a leg;
- A means for fitting a foot, the means having a longitudinal direction being aligned with the longitudinal direction (A*) of the foot, the means having a first side and a second side in longitudinal direction;
- A joint for being located behind the heel
- a first coupling means which couples the cuff to the joint
- a second coupling means which couples the means for fitting a foot to the joint;
- an ankle-cuff to be fit to a leg above the ankle and located between joint and the cuff for being fit to the lower part of a leg, the ankle-cuff being coupled to the first coupling means;
- at least a first elastic tensioning means, the at least first elastic tensioning means coupling the means for fitting a foot to the ankle-cuff.
According to some embodiments of the present invention, the ankle-cuff may have coupled to the first coupling means, the ankle-cuff having a first side to be located at one side of a leg, the ankle-cuff having a second side to be located at the other side of a leg. According to some embodiments of the present invention, the dynamic ankle orthesis may comprise a first and a second elastic tensioning means, the first elastic tensioning means coupling the first side of the means for fitting a foot to the first side of the ankle-cuff, the second elastic tensioning means coupling the second side of the means for fitting a foot to the second side of the ankle-cuff.

Optionally, each of the elastic tensioning means may comprise a elliptic-shaped elastic tensor and a first and a second coupling means for providing tension to the tensor in a direction along the long axis of the ellipse.

The elastic tensioning means may comprise at least one extension limiting means, such as a strap, e.g. a nylon or polymer strip, coupled to a first and a second coupling means for limiting the dilatation of first and a second coupling means. The limitation of the dilatation of the first and a second coupling means allows limiting the forward flexion of the foot, i.e. the stretching of the ankle.

The dynamic ankle orthesis according to the second aspect of the present invention, has the function of the leg-part of the orthesis divided into two separate parts. The first part is provided by the cuff, which is to fit the orthesis to the distal end of the leg, more particular to the calf of the leg. Optionally the cuff is located vertically moveable in the first coupling means, allowing to compensate for the so-called "pumping effect".

A second cuff, i.e. the ankle-cuff being coupled to the cuff by means of the first coupling means, and is to provide suitable arrangements to allow the joint to move. The joint is located between the ankle-cuff and the means for fitting a foot. The ankle-cuff is coupled to the means for fitting a foot via the joint and the second coupling means. The ankle-cuff is coupled to the means for fitting a foot by the at least first elastic tensioning means.

As such, the coupling of the orthesis to the distal part of the leg, more particular to the calf of the leg, and the provision of rotation possibilities to the foot, are effected by two distinguishable parts of the orthesis. This allows to control both parts in a more independent way, i.e. control one relatively independent from the other.

According to some embodiments of the present invention, the joint may rotate about at least one axis of rotation and which least one axis of rotation being aligned with one of
- the longitudinal direction (A*) of the means for fitting a foot,
- the direction (C*) of the vertical axis of rotation of the leg and
- the direction (B*) perpendicular to the plane defined by the longitudinal direction (A*) of the means for fitting a foot and the direction of the vertical axis of rotation of the leg. This direction B* is the direction of the axis of rotation of the ankle.

According to some embodiments of the present invention, the joint (3) may rotate about two of these three axes of rotation.

According to some embodiments of the present invention, the joint (3) may rotate about a first, a second and a third axis of rotation. The first axis of rotation is aligned with the longitudinal direction (A*) of the means for fitting a foot. The second axis of rotation is aligned with the direction (C*) of the vertical axis of rotation of the leg. The third axis of rotation is aligned with the direction (B*) perpendicular to the plane defined by the longitudinal direction (A*) of the means for fitting a foot and the direction of the vertical axis of rotation of the leg.

The provision of two distinguishable parts, the settable and adjustable rotation angles and optionally the provision of a plurality of axes of rotation particularly allows the harmonization of the different phases in making a step. This harmonization of the different consecutive phases improves the stability of the patient and has a direct influence on the dynamic behavior of the whole body of the patient.

According to some embodiments of the present invention, the angle of rotation about at least one, and optionally about all of the axes of rotation may be limited.

According to some embodiments of the present invention, the elastic tensioning means further may comprise at least one abutment located between a first and a second coupling means for limiting the approximation of first and a second coupling means. The abutments may optionally be adjustable. The limitation of the elastic contracting of the elastic tensioning means allows to adjust the backwards flexion of the foot, i.e. the bending of the ankle.

According to some embodiments of the present invention, the cuff may be vertically moveably coupled to the first coupling means. This may partially compensate the 'pumping' effect of the orthesis when mounted and used by a patient.

According to embodiments of the present invention, the dynamic ankle ortheses may further comprise a retractable calcanean shell allowing an easy setting in and insuring a good support of the heel. It allows also to maintain contact between heel part of the sole of the foot with the means for fitting the foot during use of the orthesis. According to embodiments of the present invention, the first coupling means may comprise at least two posts. The coupling means is coupled to the cuff by means of these at least two posts. These at least two posts may merge at the base part of the coupling means, providing a common base for being coupled to the joint.

According to embodiments of the present invention, the first coupling means, e.g. comprising at least two posts, may be slidingly mounted in a sheath at the back of the cuff. This slidingly mounted coupling of the sheath and the first coupling means, e.g. by means of at least two posts, may allow a vertical displacement of the cuff and optionally a rotation of the first coupling means.

The term "back" of the cuff is to be understood as the part of the cuff, which is adapted to receive the calf of the patient's leg. The "back part" may be understood as the part of the cuff located behind the plane defined by the vertical axis of rotation of the leg and the axis of rotation of the ankle.

According to embodiments of the present invention, the means for fitting a foot and/or the cuff and/or the first coupling means may be detachable for easy adaptation and adjusting of the orthesis to the requirements of the pathology of the patient.

Some of the embodiments of the present invention may overcome partially or completely some drawbacks of the dynamic ankle orthesis as known in the art.

It is understood that although references are made to a dynamic ankle orthesis, the different elements of the invention may be used for other orthopaedic apparatuses and devices.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Figure 1 shows schematically an embodiment of a dynamic ankle orthesis according to the present invention.
Figure 2 shows schematically an example of a jpint being part of the a dynamic ankle orthesis according to the present invention as shown in figure 1.
Figure 3 shows schematically details of elastic tensioning means comprises a elliptic-shaped elastic tensor being part of the dynamic ankle orthesis according to the present invention as shown in figure 1.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under, in front of, behind and the like in the description and the claims are used for descriptive purposes and not necessarily for describing relative positions. It is to be understood that the terms so used are interchangeable under appropriate circumstances and that the embodiments of the invention described herein may be capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only, except where explicitly stated otherwise. Thus, the scope of the expression "a device A coupled to a device B" should not be limited to devices or systems wherein one piece of device A is directly connected to a piece of device B. It means that there exists a path between the piece of A and the piece of B which may be a path including other devices or means.

The following terms are provided solely to aid in the understanding of the invention.

The term "foot" means the foot of a human patient or a part of an animal which articulates by means of an articulation rotatable around 3 axes.

The term "heel" is to be understood as the heel part of the foot, i.e. the part is below the axis of the ankle and behind the plane defined by the ankle axis and the vertical axis of the leg, behind meaning in a direction away from the toes.

The invention will now be described by a detailed description of several embodiments of the invention. It is clear that other embodiments of the invention are exemplary and can be configured according to the knowledge of persons skilled in the art without departing from the true spirit or technical teaching of the invention, the invention being limited only by the terms of the appended claims.

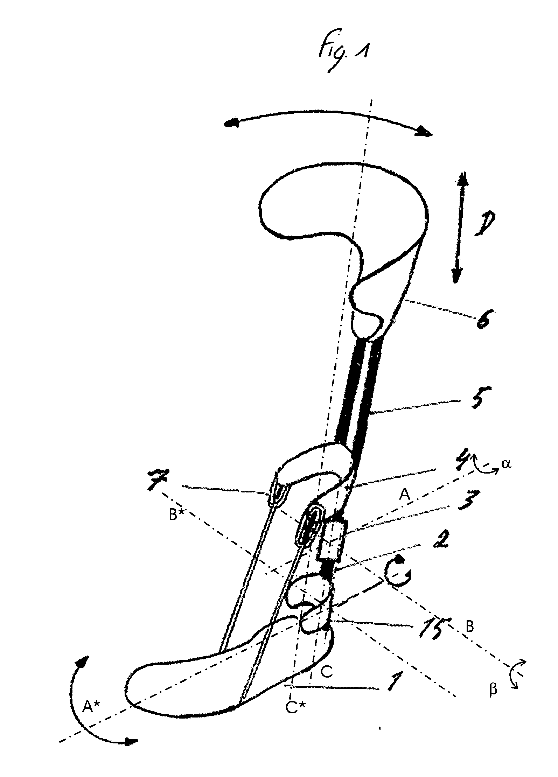

A dynamic orthesis as subject of the present invention will be described hereinafter. Reference is made to three axes of rotation. The three axes of rotation are shown in Fig. 1. The axis "A" is an axis parallel to the longitudinal axis (A*) of the foot, e.g. a foot of a human patient. The axis "B" is an axis parallel to the axis B* of the ankle. The axis "C" is a vertical axis, being parallel to the axis C* of rotation of the leg, around which the foot may be rotated.

As shown in Fig. 1, means for allowing a displacement "D" in vertical direction between a cuff 6 and a first coupling means 5, e.g. being one or more posts, may be provided.

As shown in Figure 1, the dynamic ankle orthesis comprises a cuff 6 for being fit to the lower part of a leg.

The dynamic ankle orthesis comprises a means 1 for fitting a foot having a longitudinal direction being aligned with the longitudinal direction (A*) of the foot; in this embodiment the means 1 is a shoe-inlay. The shoe inlay is optionally provided from any suitable material, which is e.g. strong enough to resist forces exercised. As an example, the shoe insert may be provided from thermoformeable material or laminated material. The shoe insert, and more in general the means for fitting the foot, has the function to fit and guide the foot wearing the orthesis.

The dynamic ankle orthesis comprises a joint 3 for being located behind the heel.

The dynamic ankle orthesis comprises a first coupling means 5, which couples the cuff to the joint. The first coupling means in this particular embodiment comprises two posts, which may have a common base, coupling the first coupling means to the joint. The possibility to vary the section, the exact form and the number of posts 3 of the coupling means may change the degree of assistance and control given to the ankle.

In particular, as will be set out further, in case the orthesis comprises a joint allowed to rotate about an axis B parallel to the axis of ankle B*, assistance and control may be given to bending and stretching of the ankle depending on the form, section and number of posts. By bending the posts 3, the balance point of stretching or bending around the axis of the ankle (B*) may be set, which enables the orthesis to be adapted according to the spasticity and muscle tonicity of the foot of the patient.

The upper part of the posts, or more in general the upper part of the first coupling means 5, are mounted slidingly in vertical direction, e.g. in a sheath located at the back of the cuff 6, allowing a displacement "D".

The latter is to limit the pumping effect of the orthesis. The slidingly mounted coupling of the upper part of the first coupling means, such as e.g. the posts, and the cuff, e.g. by means of the sheath, allows a vertical displacement of the cuff 6 and may allow rotation of upper part of the first coupling means, e.g. the posts, rotating around their individual axes. A slight pressure on the surfaces of the sheath avoids some or any extensive pumping of the orthesis. In order to avoid the cuff to slide down relative to the first coupling means under normal gravitation, the inner surface of the sheath and/or the part of the first coupling means inserted in the sheath may be covered with velvet-like material. The height of the front and rear bearing surfaces of the sheath may be different to provide different support during flexion or stretching action of the orthesis.

The first coupling means 5 may be formed from any suitable rigid material, e.g. rigid metallic or composite materials. In an alternative the first coupling means may be a resilient coupling means. In case of a plurality of posts providing the first coupling means 5, the posts may have one common base at their lower part, or may be present as separate posts. The plurality, e.g. two, posts may be replaced by one support, which increases the rigidity around the vertical axis (C*) of the leg. The posts may be unitary pieces, i.e. provided in only one part, having a given thickness, or they may be provided as a stack of several layers, each layer having its own thickness and the plurality of layers not all having the same dimensions.

The dynamic ankle orthesis comprises a second coupling means 2, being a resilient or springy means, which couples the means 1 for fitting a foot to the joint 3. The second coupling means 2 may be formed from any suitable resilient or springy material, e.g. metallic or composite materials.

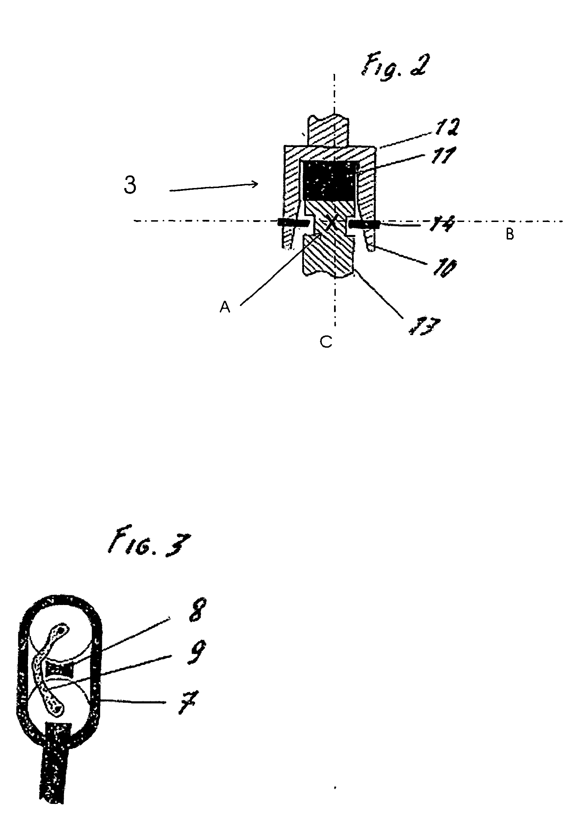

The joint 3 comprises a first axis of rotation (C), which axis is substantially parallel to the vertical axis C* of the leg of the patient wearing the orthesis. The joint, as more in detail shown in Figure 2, comprises
o a cylindrical housing 12 having an upper side and a lower side,. The lower side is provided with an aperture. The housing has an inner surface. The housing has a cylinder axis which is aligned with the first axis of rotation. The inner surface of the cylindrical housing is provided with a conical shape having its widest diameter at the lower surface;
o a bar 13 having a substantially cylindrical first outer end, the first outer end of the bar being rotatably mounted in the cylindrical housing via the aperture. The mounting allows rotation of the bar around the first axis (C) of rotation. The housing has an inner surface with a conical shape 10. The conical shape allows the bar to swing over an angle β around at least the second axis of rotation A, which second axis of rotation is aligned with the longitudinal direction of the means for fitting a foot.

The joint 3 is located behind the heel part of the foot. The conical shape of the inner surface of the housing further may allow a rotation about a third axis B, being an axis substantially parallel to the axis of the ankle B*. In the embodiment as shown in Figure 1, a set of setscrews 14 limits the rotation about the third axis over an angle y. In this particular embodiment angle β is smaller than angle y.

The housing 12 is connected to the leg-part of the orthesis, in particular to the first coupling means 5. The bar is connected to the second coupling means 2 .

The joint 3 as shown in Figure 2, further comprises a rotation limiting means, in particular in this embodiment a flexible sleeve or socket 11 located inside the cylindrical housing. The presence of this flexible sleeve or socket 11 prevents the bar to rotate when the sleeve 11 is compressed between the housing and the upper surface of the bar, e.g. compressed in the direction of the axis of the cylindrical housing. As an alternative, the sleeve may be a flexible disc, functioning in the same way.

The bar may comprise a recess along the outer surface of the cylindrical first outer end for receiving the setscrews 14. The setscrews 14, which may extend through the housing 12 may be set so they enter into the recess, which may prevent the bar to move out of the housing by displacing in a direction parallel to axis C.

One or more setscrews may be used to limit the amplitude of rotation about one of the axes A or B, or for both axes A and B. It is understood a plurality of setscrews may be provided.

A rotation about the axis "A", being an axis parallel to the longitudinal axis (A*) of the foot, is optionally limited between -15° and 15°, wherein 0°means that the foot is oriented according to its normal position, i.e. the sole being substantially parallel to the horizontal plane. A positive angle means a pronation of the foot, a negative angle means a supination of the foot. A rotation about the axis "B", being an axis parallel to the axis B* of the ankle, is optionally limited between -20° and 45°, wherein 0° means that the foot and ankle are oriented in its normal position, i.e. with the foot having its sole substantially parallel to the horizontal plane. A positive angle means a stretching of the ankle, i.e. a rotation downwards of the front side of the foot, away from the leg. A negative angle means a bending of the ankle, i.e. a rotation upwards of the front side of the foot, towards the leg. The rotation about the axis "C" may also be limited. The rotation about axis C, being an axis parallel to the axis C^{*} of rotation of the leg, around which the foot may be rotated the axis "C" may also be limited to -15° to +15°. 0° means that the foot is oriented in its normal position, i.e. with the toes pointing forwards. A positive angle means a rotation of the first foot in a direction away from the other foot. A negative angle means a rotation of the first foot in a direction towards the other foot.

As shown in Figure 1, the dynamic ankle orthesis may comprise an ankle-cuff 4. The ankle-cuff 4 is coupled to the first coupling means 5, optionally the ankle-cuff is connected directly to the housing 12 of the joint 3.

The dynamic ankle orthesis further may comprise at least a first elastic tensioning means, in this particular embodiment two, i.e. a first and a second elastic tensioning means. The first and second elastic tensioning means couple the means for fitting a foot to the ankle-cuff. Each of the elastic tensioning means comprise an elliptic-shaped elastic tensor 7 and a first and a second coupling means for providing tension to the tensor in a direction along the long axis of the ellipse. The elliptic-shaped elastic tensor is optionally provided at the upper part of the elastic tensioning means, i.e. near coupling of the elastic tensioning means to the ankle-cuff. The lower part of the elastic tensioning means is connected to the means 1 for fitting the foot.

The ankle-cuff has a first side for being located at one side of a leg when the orthesis is worn. The ankle-cuff has a second side to be located at the other side of a leg.

The first elastic tensioning means couples a first longitudinal side of the means for fitting a foot to the first side of the ankle-cuff. The second elastic tensioning means couples the second longitudinal side of the means for fitting a foot to the second side of the ankle-cuff.

Each of the elastic tensioning means comprises at least one extension limiting means, such as a strap 9 coupled to a first and a second coupling means for limiting the dilatation of first and a second coupling means. This extension limiting means limits the degree of stretching of the ankle, hence for controlling, even avoiding hyper tensioning of the knee.

The elastic tensioning means further comprises at least one abutment 8 located between a first and a second coupling means for limiting the approximation of first and a second coupling means. This has as an effect that, the contraction of the elastic tensors is limited. Once the ankle is bend to a given and set angle, no force is applied anymore to the sole of the foot, in order to correct the inclination of the ankle. Avoiding such force provides comfort to the patient wearing the orthesis.

In the embodiment shown in Fig. 3, the elastic tensioning means is provided with a substantially elliptic elastic and a tape 9. It is understood that also alternative means for providing tension may be used, such as springs.

Optionally the dynamic ankle orthesis may comprise a calcanean shell 15, which allows easy and correctly mounting the orthesis to the leg of a patient.

In use of the dynamic ankle orthesis provides harmonization of the different consecutive phases improves the stability of the patient and has a direct influence on the dynamic behavior of the whole body of the patient.

Under normal walking action, during the swing phase, the foot is held in dorsal flexion. When the foot contacts the ground, the heel contacts the ground first, which starts a bending or flexion of the knee. It prepares the toe-off phase.

In case a patient suffers from a spastic or pending foot, during the pending phase, the front part of the foot tends to remain in contact with the ground, which may cause the person to swing his hip or swing his leg sidewise. The front part of the foot makes contact with the ground first. The knee will not be pushed forwards, i.e. start to flex, which will cause the knee to remain or to come into hyperextension. The toe-off phase will largely be changed.

This pathology may cause frequently an inward orientation of a.o. the knee and/or a luxation of the ankle, i.e. dislocation of an ankle.

When the dynamic ankle orthesis according to the present invention is used, the orthesis assists to move the foot, while the a sufficient liberty to move the foot is provided, due to the rotation of the joint in one or even according to three axes of rotation, due to the elastic tensioning means and/or the system of vertical movement of the cuff about the first coupling means.

The elastic tensioning means may be adjusted to compensate and reequilibrate the deficiencies in forces applied by the muscles causing the ankle to stretch or bend. The ankle-cuff, and the provision of the elastic tensioning means may allow to apply a force to the lower part of the leg and feet, to compensate exaggerated pronation or supination. The sufficient liberty to move the foot is provided in particular in case of 3D articulation of the joint, i.e. rotation about 3 axes, optionally facilitated by the possibility of vertical displacement of the cuff.

It is to be understood that although preferred embodiments, specific constructions and configurations, as well as materials, have been discussed herein for devices according to the present invention, various changes or modifications in form and detail may be made without departing from the scope and spirit of this invention.

## Claims

1. dynamic ankle orthesis comprising
- a cuff (6) suitable for receiving the calf of the leg of a patient;
- A means (1) for fitting a foot;
- A joint comprising a first axis of rotation (C), the joint comprising
o a cylindrical housing (12) having an upper side and a lower side, the lower side being provided with an aperture, the housing having an inner surface, the housing having an cylinder axis which is aligned with the first axis of rotation,
o a bar (13) having a substantially cylindrical first outer end, the first outer end of the bar being rotatably mounted in the cylindrical housing via the aperture, the mounting allowing rotation of the bar around the first axis (C) of rotation
- a first coupling means (5), which couples the cuff to the upper side of the cylindrical housing
- a second coupling means (2) which couples the means for fitting a foot to the bar
**characterized in that** the the inner surface of said cylindrical housing is provided with a conical shape having its widest diameter at the lower surface, said conical shape allowing the bar to swing over an angle β around at least a second axis of rotation (A).

2. A dynamic ankle orthesis according to claim 1, wherein the means (1) for fitting a foot has a longitudinal direction being aligned with the longitudinal direction (A*) of the foot, the second axis (A) of rotation is aligned with the longitudinal direction (A*) of the means for fitting a foot.

3. A dynamic ankle orthesis according to any one of the claims 1 to 2, wherein the joint further comprises a third axis of rotation (B), which third axis of rotation is perpendicular to the plane defined by the first axis of rotation and the second axis of rotation, the joint comprises means to allow rotation of the bar around the third axis of rotation over an angle γ.

4. A dynamic ankle orthesis according to claim any one of the claims 1 to 3, wherein at least one of the angles β or γ can be set by means of at least one setscrew (14).

5. A dynamic ankle orthesis according to any one of the claims 1 to 4, wherein the at least one setscrew extends through the cylindrical housing.

6. A dynamic ankle orthesis according to any one of the claims 1 to 4, wherein the dynamic ankle orthesis comprises a ankle-cuff (4) to be fit to a leg above the ankle and located between joint and the cuff for being fit to the lower part of a leg, the ankle-cuff being coupled to the first coupling means, the dynamic ankle orthesis comprising at least a first elastic tensioning means, the at least the first elastic tensioning means coupling the means for fitting a foot to the ankle-cuff.

7. A dynamic ankle orthesis according to claim 6, wherein the means for fitting a foot (1) having a first side and a second side in longitudinal direction, the ankle-cuff having a first side to be located at one side of a leg, the ankle-cuff having a second side to be located at the other side of a leg, the orthesis comprising a first and a second elastic tensioning means, the first elastic tensioning means coupling the first side of the means for fitting a foot to the first side of the ankle-cuff, the second elastic tensioning means coupling the second side of the means for fitting a foot to the second side of the ankle-cuff.

8. A dynamic ankle orthesis according to claim 7, wherein each of the elastic tensioning means comprises a elliptic-shaped elastic tensor (7) and a first and a second coupling means for providing tension to the tensor in a direction along the long axis of the ellipse, the elastic tensioning means comprises at least one extension limiting means (9) coupled to a first and a second coupling means for limiting the dilatation of first and a second coupling means.

9. A dynamic ankle orthesis according to any one of the claims 6 to 8, wherein each of the elastic tensioning means further comprise at least one abutment (8) located between a first and a second coupling means for limiting the approximation of first and a second coupling means.

10. A dynamic ankle orthesis comprising:
- A cuff (6) for being fit to the lower part of a leg;
- A means (1) for fitting a foot, the means having a longitudinal direction being aligned with the longitudinal direction (A*) of the foot, the means (1) having a first side and a second side in longitudinal direction;
- A joint (3) for being located behind the heel
- a first coupling means (5), which couples the cuff to the joint
- a second coupling means (2) which couples the means for fitting a foot to the joint;
- an ankle-cuff (4) to be fit to a leg above the ankle and located between joint and the cuff for being fit to the lower part of a leg, the ankle-cuff being coupled to the first coupling means;
- at least a first elastic tensioning means, the at least first elastic tensioning means coupling the means for fitting a foot to the ankle-cuff.

11. A dynamic ankle orthesis according to claim 10, wherein the ankle-cuff having a first side to be located at one side of a leg, the ankle-cuff having a second side to be located at the other side of a leg, the dynamic ankle orthesis comprising a first and a second elastic tensioning means, the first elastic tensioning means coupling the first side of the means for fitting a foot to the first side of the ankle-cuff, the second elastic tensioning means coupling the second side of the means for fitting a foot to the second side of the ankle-cuff.

12. A dynamic ankle orthesis according to claim 11, wherein each of the elastic tensioning means comprises a elliptic-shaped elastic tensor (7) and a first and a second coupling means for providing tension to the tensor in a direction along the long axis of the ellipse, the elastic tensioning means comprises at least one extension limiting means (9) coupled to a first and a second coupling means for limiting the dilatation of first and a second coupling means.

13. A dynamic ankle orthesis according to any one of the claims 11 to 12, wherein the joint (3) may rotate about at least one axis of rotation, the at least one axis of rotation being aligned with one of the longitudinal direction (A*) of the means for fitting a foot, the direction of the vertical axis (C*) of rotation of the leg and the direction (B*) perpendicular to the plane defined by the longitudinal direction (A*) of the means for fitting a foot and the direction of the vertical axis (C*) of rotation of the leg.

14. A dynamic ankle orthesis according to claim 13, wherein the joint (3) may rotate about a first, a second and a third axis of rotation, the first axis (A) of rotation being aligned with the longitudinal direction (A*) of the means for fitting a foot, the second axis (C) of rotation being aligned with the direction (C*) of the vertical axis of rotation of the leg and the third axis (B) of rotation being aligned with the direction (B*) perpendicular to the plane defined by the longitudinal direction (A*) of the means for fitting a foot and the direction of the vertical axis of rotation of the leg.

15. A dynamic ankle orthesis according to any one of the claims 13 or 14, wherein the angle of rotation about at least one of the axis of rotation is limited.

16. A dynamic ankle orthesis according to any one of the claims 11 to 15, wherein each of the elastic tensioning means further comprise at least one abutment (8) located between a first and a second coupling means for limiting the approximation of first and a second coupling means

17. A dynamic ankle orthesis according to any one of the claims 1 to 16, wherein the cuff is vertically moveably coupled to the first coupling means (5).
